# EUROPEAN PATENT APPLICATION

(11) **EP 4 807 756 A1**
(43) Date of publication of application: **16.09.2026**
(21) Application number: 25226113.6
(22) Date of filing: 21.12.2025
(51) Int. Cl.: G16H 20/60

(54) **AI BASED RECIPE GENERATION BY INTEGRATING INGREDIENT PAIRING SCORE DETERMINED FROM FLAVOR SPACES**

(30) Priority: 12.03.2025 IN 202521022285
(71) Applicant: Tata Consultancy Services Limited, Mumbai, Maharashtra 400 021 (IN)
(72) Inventor: PANDE, Harshvardhan, 411057 Pune (IN); KAUSLEY, Shankar Balajirao, 411057 Pune (IN); RAI, Beena, Pittsburgh, 15213 (US)
(74) Representative: Goddar, Heinz J.

(57) **Abstract**

A method and system for Al based recipe generation by integrating ingredient pairing score determined from flavor spaces is disclosed. Current available recipe generation systems do not focus on creating recipes optimized for the specific nutritional needs associated with various health conditions. The method disclosed incorporates nutritional and flavor profiles for pairing suitable ingredients and generating recipes. The method identifies a primary ingredient based on conditional nutrient-based filtering technique. Based on the selected primary ingredient the method employs flavor compound-based embeddings to assess flavor compatibility between this primary ingredient and other potential ingredients, identifying suitable secondary ingredients for the recipe. The secondary ingredients are determined by determining ingredient pairing score using directional similarity measure, orientation factor and spatial proximity measure from molecular embedding matrices represented as flavor spaces of the primary ingredient and the secondary ingredients. The method generates personalized recipes used to manage various lifestyle related diseases.

## Description

### CROSS-REFERENCE TO RELATED APPLICATIONS AND PRIORITY

The present application claims priority to Indian application no. 202521022285, filed on March 12, 2025.

### TECHNICAL FIELD

The disclosure herein generally relates to artificial intelligence (AI) based recipe generation, and, more particularly, to a method and system for AI based recipe generation by integrating ingredient pairing score determined from flavor spaces.

### BACKGROUND

The prevalence of lifestyle diseases such as diabetes, hypertension, high cholesterol, and various other metabolic conditions has surged due to increasingly sedentary lifestyles and poor dietary habits. Traditionally, these diseases are managed with medications prescribed by healthcare professionals. However, these medications often come with a range of side effects. This has led to a growing interest in dietary interventions as a complementary or alternative approach to manage these conditions. Dietary approaches such as the Dietary Approaches to Stop Hypertension (DASH) and Mediterranean diet (MedDiet) and inclusion of superfoods like vegetables, millet and legumes have demonstrated efficacy in managing various lifestyle-related conditions, including hypertension, diabetes, and cardiovascular diseases. Creating diet plans and recipes to target lifestyle diseases requires expertise across multiple domains, including food science, nutritional science, and culinary arts. Integrating knowledge from these areas to generate effective recipes is complex. Even domain experts must often review extensive information to create novel and tailored recipes.

With rapid technological advancements and the rise of artificial intelligence (AI), access to information has become more convenient and immediate for users. Traditional recipe databases and cookbooks have largely been replaced by mobile applications that provide instant access to a wide variety of recipes. A particularly impactful development in this space has been the integration of generative AI, which leverages vast databases of recipes to create AI-driven culinary assistants. However, current AI-based recipe recommendation systems lack critical nuances in nutritional and dietary science and culinary arts.

The field of recipe recommendation using artificial intelligence and ingredient pairing techniques has been researched extensively in the past. Text generation has been at the forefront of generative AI. Recent advancements in large language models have enabled the exploration of tasks such as recipe generation. The availability of extensive recipe databases, such as Recipe1M+ and RecipeNLG, which comprise over 2 million recipes and ingredient information, has led to increased interest in this area. One prior method developed Food Lens, a smart system that identifies food classes and generates relevant recipes from images using a Convolutional Neural Network architecture. Another prior method employed generative AI tools, including ChatGPT and other fine-tuned models, for recipe generation. Yet another prior method introduced Smart Cuisine, a generative AI-powered nutrition assistant designed for sustainable cooking, aiming to optimize ingredient usage and minimize food waste during preparation. In another prior method a multi-modal model integrates visual food embeddings with recipe prompts, thereby enhancing the quality of recipe generation and domain-specific comprehension. Another prior method proposed SHARE, a hierarchical system for assistive recipe editing that substitutes ingredient and generates natural language cooking steps. These current recipe recommendation systems do not focus on creating recipes optimized for the specific nutritional needs associated with various health conditions.

### SUMMARY

Embodiments of the present disclosure present technological improvements as solutions to one or more of the above-mentioned technical problems recognized by the inventors in conventional systems. For example, in one embodiment, a method for artificial intelligence (AI) based recipe generation by integrating ingredient pairing score determined from flavor spaces is provided. The method includes determining a set of ingredients from a plurality of ingredients based on a set of macronutrient profiles suitable for one or more medical conditions of a user. Furthermore, the method includes determining a set of secondary ingredients from the set of ingredients by evaluating an ingredient compatibility between each ingredient amongst the set of ingredients with a primary ingredient selected from the set of ingredients. The ingredient compatibility is evaluated based on an ingredient pairing score calculated for each ingredient amongst the set of ingredients using a directional alignment measure, an orientation factor, and a spatial proximity measure determined from a molecular embedding matrix represented as a flavor space. The method finally includes generating a recipe comprising a set of cooking instructions from the primary ingredient and the set of secondary ingredients using a text generation model fine-tuned on a recipe dataset based on an optimal token length estimated from an optimal token count per cooking instruction.

In another aspect, a system for AI based recipe generation by integrating ingredient pairing score determined from flavor spaces is provided. The system comprises memory storing instructions; one or more communication interfaces; and one or more hardware processors coupled to the memory via the one or more communication interfaces, wherein the one or more hardware processors are configured by the instructions to determine a set of ingredients from a plurality of ingredients based on a set of macronutrient profiles suitable for one or more medical conditions of a user. Furthermore, the system includes determining a set of secondary ingredients from the set of ingredients by evaluating an ingredient compatibility between each ingredient amongst the set of ingredients with a primary ingredient selected from the set of ingredients. The ingredient compatibility is evaluated based on an ingredient pairing score calculated for each ingredient amongst the set of ingredients using a directional alignment measure, an orientation factor, and a spatial proximity measure determined from a molecular embedding matrix represented as a flavor space. The system finally includes generating a recipe comprising a set of cooking instructions from the primary ingredient and the set of secondary ingredients using a text generation model fine-tuned on a recipe dataset based on an optimal token length estimated from an optimal token count per cooking instruction.

The set of secondary ingredients are determined from the set of ingredients by converting a first set of flavor compounds corresponding to the primary ingredient to a first molecular embedding matrix representing a first flavor space using a pre-trained molecular property prediction model. Then the ingredient compatibility is evaluated between the primary ingredient and each ingredient amongst the set of ingredients by determining the ingredient pairing score. Next, the set of secondary ingredients from the set of ingredients are selected based on the ingredient pairing score between each ingredient amongst the set of ingredients and the primary ingredient.

The ingredient pairing score between the primary ingredient and an ingredient amongst the set of ingredients is determined by converting a second set of flavor compounds corresponding to the ingredient to a second molecular embedding matrix representing a second flavor space using the pre-trained molecular property prediction model. Then the directional similarity measure is computed utilizing a principal angle computed between the first flavor space and the second flavor space. Next the orientation factor is computed between the flavor space and the second flavor space utilizing a set of singular vectors determined by performing singular value decomposition of a matrix obtained from the first molecular embedding matrix and the second molecular embedding matrix. Then, the spatial proximity measure is computed based on a Euclidean distance measure between a first centroid corresponding to the first flavor space and a second centroid corresponding to the second flavor space. Finally, the ingredient pairing score is determined based on the directional similarity measure, the orientation factor, and the spatial proximity measure.

The ingredient pairing score is mathematically represented as, $Ingredient pairing score = \frac{λ . cos θ}{1 + d}$ where cos *θ* represents the directional similarity measure, *λ* represents the orientation factor and d represents the spatial proximity measure.

In yet another aspect, there is provided a computer program product comprising a non-transitory computer readable medium having a computer readable program embodied therein, wherein the computer readable program, when executed on a computing device causes the computing device for AI based recipe generation by integrating ingredient pairing score determined from flavor spaces is provided. The computer readable program includes determining a set of ingredients from a plurality of ingredients based on a set of macronutrient profiles suitable for one or more medical conditions of a user. Furthermore, the computer readable program includes determining a set of secondary ingredients from the set of ingredients by evaluating an ingredient compatibility between each ingredient amongst the set of ingredients with a primary ingredient selected from the set of ingredients. The ingredient compatibility is evaluated based on an ingredient pairing score calculated for each ingredient amongst the set of ingredients using a directional alignment measure, an orientation factor, and a spatial proximity measure determined from a molecular embedding matrix represented as a flavor space. The computer readable program finally includes generating a recipe comprising a set of cooking instructions from the primary ingredient and the set of secondary ingredients using a text generation model fine-tuned on a recipe dataset based on an optimal token length estimated from an optimal token count per cooking instruction.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 illustrates an exemplary block diagram of a system for Artificial Intelligence (AI) based recipe generation by integrating ingredient pairing score determined from flavor spaces according to some embodiments of the present disclosure.
FIG.2 is an exemplary flow diagram illustrating a method for AI based recipe generation by integrating ingredient pairing score determined from flavor spaces according to some embodiments of the present disclosure.
FIG.3 illustrates an overall block diagram depicting the method for AI based recipe generation by integrating ingredient pairing score determined from flavor spaces according to some embodiments of the present disclosure.
FIG.4 illustrates a flavor space representation of ingredients in three-dimensional space according to some embodiments of the present disclosure.
FIG.5 illustrates the distribution of the number of cooking instructions within a dataset according to some embodiments of the present disclosure.
FIG.6 illustrates the distribution of average cooking instruction length in the dataset according to some embodiments of the present disclosure.
FIG.7 illustrates flavor compatibility scale representing similar and contrasting flavor profiles according to some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Artificial Intelligence (AI) based food recipe recommendation systems leverage the power of large-language models to generate diverse and personalized recipes. However, the diverse dietary preferences influenced by health conditions or personal tastes demand a high degree of personalization to address the user's nuanced requirements. Despite using novel techniques such as generative AI, existing recipe recommendation systems fall short in integrating critical aspects, such as nutritional profiling and flavor aspects of ingredients. This limits these systems from creating recipes tailored for lifestyle diseases with choice of ingredient based on user preference. A significant limitation of current AI-based recipe recommendation systems is that they lack incorporated ingredient pairing into the recipe generation process. As ingredient pairing is one of the most important aspects of culinary arts, absence of this aspect decreases the suitability and lacks the personalization feature from these tools. The lack of these nuances reduces the perceived trustworthiness of the recipes generated by AI, potentially making them less appealing and reliable for consumers.

Embodiments of the present disclosure address the above said challenges through a method which integrates ingredient selection (based on nutritional and flavor profile) with AI-based recipe recommendation systems to enable personalized recommendations tailored to individual health needs and taste preferences. The method disclosed uses higher-dimensional embeddings to represent flavor compounds present in various ingredients and develop a unique technique named "flavor space" to examine compatibility between various ingredients based on flavor profile. Finally, fine-tuned large language models are used for recipe generation tasks.

The disclosed method includes two main modules such as an ingredient selection module and a recipe generation module. To account for both nutritional value and flavor profile, the ingredient selection module comprises two sub-modules: an ingredient filtering sub-module and an ingredient pairing sub-module. In the ingredient filtering sub-module, conditional nutrient-based filtering technique is applied to identify a primary or main ingredient for developing the recipe. These conditions are based on relevant nutritional criteria that align with the dietary needs of the specific lifestyle disorder. Based on the primary ingredient selected in the ingredient filtering sub-module, the ingredient pairing sub-module employs flavor compound-based embeddings to assess flavor compatibility between this main ingredient and other potential ingredients, identifying suitable secondary ingredients for the recipe. The ingredient selection module then outputs a refined ingredient set to a recipe generation module. Here, a fine-tuned text generation model trained on a recipe dataset is used to generate a complete recipe based on the selected ingredients.

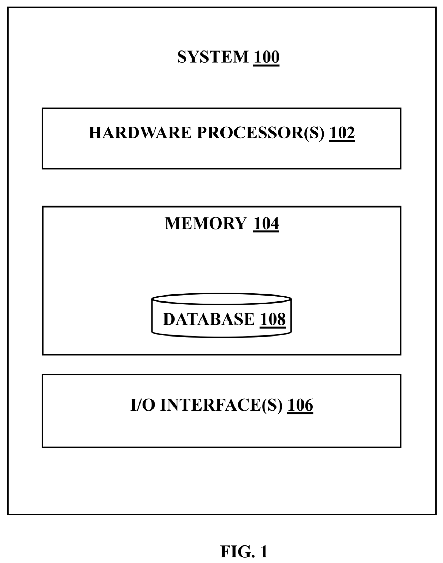

Referring now to the drawings, and more particularly to FIG. 1 through FIG. 7, where similar reference characters denote corresponding features consistently throughout the figures, there are shown preferred embodiments and these embodiments are described in the context of the following exemplary system and/or method.

FIG. 1 illustrates an exemplary block diagram of a system 100 for AI based recipe generation by integrating ingredient pairing score determined from flavor spaces according to some embodiments of the present disclosure. In an embodiment, the system 100 includes one or more hardware processors 102, communication interface(s) or input/output (I/O) interface(s) 106, and one or more data storage devices or memory 104 operatively coupled to the one or more processors 102. The one or more hardware processors 102 that are hardware processors can be implemented as one or more microprocessors, microcomputers, microcontrollers, digital signal processors, central processing units, state machines, graphics controllers, logic circuitries, and/or any devices that manipulate signals based on operational instructions. Among other capabilities, the processor(s) are configured to fetch and execute computer-readable instructions stored in the memory. In the context of the present disclosure, the expressions 'processors' and 'hardware processors' may be used interchangeably. In an embodiment, the system 100 can be implemented in a variety of computing systems, such as laptop computers, notebooks, hand-held devices, workstations, mainframe computers, servers, a network cloud and the like.

The I/O interface (s) 106 may include a variety of software and hardware interfaces, for example, a web interface, a graphical user interface, and the like and can facilitate multiple communications within a wide variety of networks and protocol types, including wired networks, for example, LAN, cable, etc., and wireless networks, such as WLAN, cellular, or satellite. In an embodiment, the I/O interface(s) can include one or more ports for connecting a number of devices to one another or to another server.

The memory 104 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes.

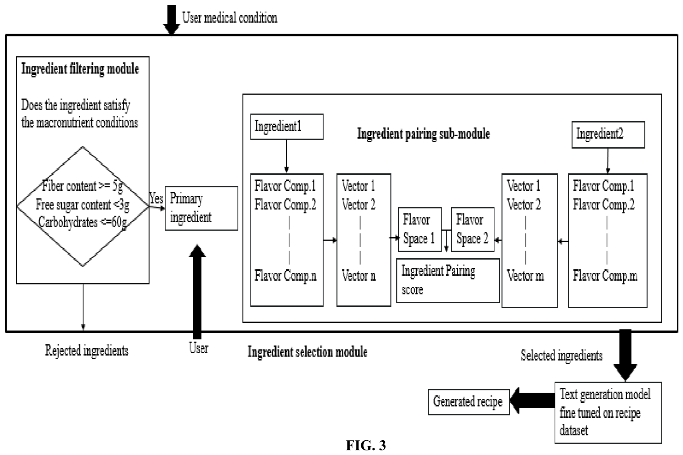

In an embodiment, the memory 104 includes a plurality of modules, such as the ingredient selection module (as depicted in FIG. 3), the recipe generation module (as depicted in FIG. 3), and the like. Further, the plurality of modules includes programs or coded instructions that supplement applications or functions performed by the system 100 for executing different steps involved in the process for AI based recipe generation by the system 100. The plurality of modules, amongst other things, can include routines, programs, objects, components, and data structures, which performs particular tasks or implement particular abstract data types. The plurality of modules may also be used as, signal processor(s), node machine(s), logic circuitries, and/or any other device or component that manipulates signals based on operational instructions. Further, the plurality of modules can be used by hardware, by computer-readable instructions executed by the one or more hardware processors 102, or by a combination thereof. The plurality of modules can include various sub-modules such as the ingredient filtering sub-module (as depicted in FIG. 3) and the ingredient pairing sub-module (as depicted in FIG. 3).

Further, the memory 104 may include a database 108 or repository. The memory 104 may comprise information pertaining to input(s)/output(s) of each step performed by the processor(s) 102 of the system 100 and methods of the present disclosure. In an embodiment, the database 108 may be external (not shown) to the system 100 and coupled via the I/O interface 106. The database may include the recipe dataset etc.

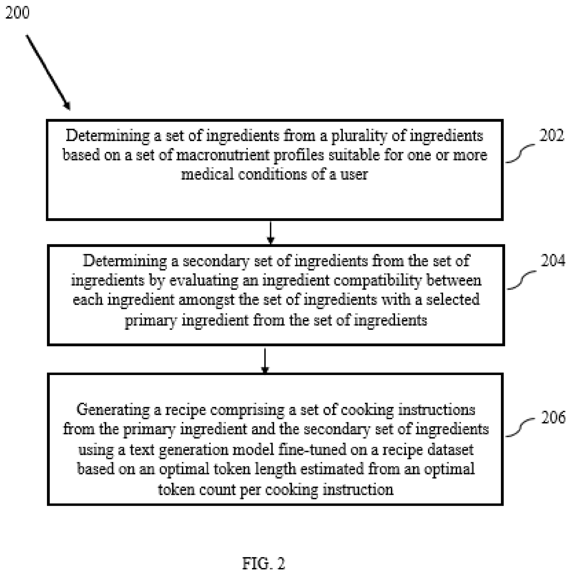

FIG. 2 is an exemplary flow diagram illustrating a method 200 for AI based recipe generation by integrating ingredient pairing score determined from flavor spaces according to some embodiments of the present disclosure. In an embodiment, the system 100 comprises one or more data storage devices or the memory 104 operatively coupled to the one or more hardware processor(s) 102 and is configured to store instructions for execution of steps of the method 200 by the processor(s) or one or more hardware processors 102. The steps of the method 200 of the present disclosure will now be explained with reference to the components or blocks of the system 100 as depicted in FIG. 1, and the steps of flow diagram as depicted in FIG. 2. The method 200 may be described in the general context of computer executable instructions. Generally, computer executable instructions can include routines, programs, objects, components, data structures, procedures, modules, functions, etc., that perform particular functions or implement particular abstract data types. The method 200 may also be practiced in a distributed computing environment where functions are performed by remote processing devices that are linked through a communication network. The order in which the method 200 is described is not intended to be construed as a limitation, and any number of the method blocks described can be combined in any order to implement the method 200, or an alternative method. Furthermore, the method 200 can be implemented using any suitable hardware, software, firmware, or combination thereof. The overall steps explained henceforth are shown as a block diagram in FIG. 3. FIG. 3 is an overall block diagram depicting the method for AI based recipe generation by integrating ingredient pairing score determined from flavor spaces according to some embodiments of the present disclosure.

Now referring to FIG. 2, at step 202 of the method 200, one or more hardware processors 102 are configured to determine a set of ingredients from a plurality of ingredients based on a set of macronutrient profiles suitable for one or more medical conditions of a user. As shown in FIG. 3, the ingredient selection module comprises the ingredient filtering sub-module and the ingredient pairing sub-module. The ingredient filtering sub-module shortlist ingredients based on macronutrient profiles which are suitable for the specified medical condition set by the user. This sub-module uses conditional nutrient-based filtering to isolate ingredients that meet the required nutritional criteria. It utilizes nutritional guidelines from various established diet plans tailored for lifestyle-related disorders, such as hypertension, diabetes, and cardiovascular diseases. For example, as shown in FIG. 3, the macronutrient conditions may be fiber content is greater than or equal to 5 gm, free sugar content is less than 3 gm, carbohydrates is less than or equal to 60 gm. If an ingredient satisfies these conditions, then it is selected as the set of ingredients from the plurality of ingredients. The resulting list of these filtered ingredients or the set of ingredients is presented to the user, allowing them to select a primary or main ingredient for the recipe. This selected primary ingredient serves as the central component for the recipe, with all secondary ingredients subsequently chosen based on their compatibility with the primary ingredient. The pairing process occurs in the subsequent module such as the ingredient pairing sub-module.

At step 204 of the method 200, one or more hardware processors 102 are configured to determine a set of secondary ingredients from the set of ingredients by evaluating an ingredient compatibility between each ingredient amongst the set of ingredients with a primary ingredient selected from the set of ingredients. The ingredient compatibility is evaluated based on an ingredient pairing score calculated for each ingredient amongst the set of ingredients using a directional alignment measure, an orientation factor, and a spatial proximity measure determined from a molecular embedding matrix represented as a flavor space. The phrases "flavor space" and "flavor subspace" and the phrases "space" and "subspace" may alternatively be used in the present disclosure. In the culinary arts, food pairings are traditionally guided by evaluating the compatibility between ingredients. This compatibility is often based on either the co-occurrence of ingredients in established recipes or the degree to which ingredients share common chemical compounds.

The set of secondary ingredients are determined by initially converting a first set of flavor compounds corresponding to the primary ingredient to a first molecular embedding matrix representing a first flavor space using a pre-trained molecular property prediction model. The method disclosed uses the pretrained molecular property prediction model optimized for molecular property prediction, to generate high-dimensional embeddings for flavor compounds corresponding to the ingredient. This representation of flavor compounds takes the molecular fingerprint into account, hence providing a better and more nuanced way to understand flavor similarity. However, the overall flavor profile of an ingredient depends on the contributions of its individual flavor compounds, which, in turn, are influenced by their respective concentrations or intensities. Measuring these concentrations typically requires costly analytical techniques, such as gas chromatography, which are not always feasible. Hence the method disclosed uses the concept of a "Flavor Space" (F), defined as the subspace spanned by the linear combination of flavor compound vectors (*vᵢ*) as represented in equation (1). $F = {\sum }_{i = 1}^{N} {a}_{i} {v}_{i}$ $Subject to {\sum }_{i = 1}^{N} {a}_{i} = 1$ Here, *aᵢ* represents the contribution of the i^{th} flavor compound to the overall flavor profile of the ingredient. By utilizing the flavor space, the challenge of obtaining precise concentrations or intensities of each flavor compound within the ingredient is addressed.

Further, for determining the set of secondary ingredients, the ingredient compatibility between the primary ingredient and each ingredient amongst the set of ingredients is evaluated. This evaluation is based on determination of the ingredient pairing score. To compute the ingredient compatibility, the ingredient pairing sub-module first generates molecular embeddings from the Simplified Molecular Input Line Entry System (SMILES) representations of flavor compounds using the pretrained molecular property prediction model. These embeddings are then projected into a higher-dimensional space of dimension d, capturing complex chemical relationships. ${v}_{i} = pretrained molecular property prediction model \left({S}_{i}\right)$ where *Sᵢ* = SMILES representation of flavor compounds.

Let V and U denote the flavor matrices for ingredients 1 and 2, respectively. And let ingredient 1 be the primary ingredient and ingredient 2 be the ingredient from the set of ingredients. A second set of flavor compounds corresponding to the ingredient amongst the set of ingredients is converted to a second molecular embedding matrix representing a second flavor space using the pre-trained molecular property prediction model. $V = {\left[\begin{matrix}{v}_{1} \\ | \\ | \\ | \\ {v}_{n}\end{matrix}\right]}_{dxn} , U = {\left[\begin{matrix}{u}_{1} \\ | \\ | \\ | \\ {u}_{m}\end{matrix}\right]}_{dxm}$

The linear combination of vectors in V and U spans a flavor space of ingredient 1 and 2 respectively. These subspaces encapsulate the various possible combinations of flavor compounds, serving as a mathematical representation of the overall flavor of each ingredient. To evaluate the compatibility between ingredients, the similarity between these flavor subspaces is computed within a high-dimensional embedding space. This similarity is evaluated based on factors such as the directional similarity measure of the flavor spaces, the spatial proximity measure and the orientation factor. To quantify the directional similarity measure, the principal angles between the flavor subspaces is computed by first calculating the transpose product V^{T}U. Then Singular Value Decomposition (SVD) is performed on the resulting n x m matrix V^{T}U, which provides the principal angles necessary to assess the directional similarity between the two flavor spaces. ${V}^{T} U = W \sum {Z}^{T}$ where W = *n* × *k* orthogonal matrix
Z = *k* × *m* orthogonal matrix $Σ = \left[\begin{matrix}{σ}_{1} & 0 & 0 & 0 & 0 \\ 0 & {σ}_{2} & 0 & 0 & 0 \\ 0 & 0 & … & 0 & 0 \\ 0 & 0 & 0 & … & 0 \\ 0 & 0 & 0 & 0 & {σ}_{k}\end{matrix}\right]$ where *k* = min (m, n)
The singular value *σᵢ* represent the cosines of the principal angles between the flavor subspaces of ingredients 1 and 2, effectively quantifying their directional alignment. To capture the minimum level of directional similarity between these flavor subspaces, we focus on, *σₘᵢₙ*, the smallest singular value. $cos θ = {σ}_{min}$ However, the principal angles between subspaces quantify directional alignment or directional similarity but do not capture their relative orientation. This limitation arises from the inherent property of singular values, which are always positive, restricting

cos *θ* to positive values and *θ* to the range 0 to $\frac{π}{2}$. Consequently, when the true angle *θ* between subspaces exceeds $\frac{π}{2}$, it is adjusted to *π* - *θ*, ensuring it remains within the constrained range. To account for orientation differences between subspaces, singular vectors obtained from singular value decomposition are utilized to formulate an orientation factor. From the singular value decomposition of matrix V^{T}U, the smallest singular value *σₘᵢₙ* is identified, and the corresponding singular vectors *qₘᵢₙ* and *pₘᵢₙ* are:
*qₘᵢₙ*: The singular vector in the column space of V, representing the direction in the subspace V, which forms an angle of *θ* with U.
*pₘᵢₙ*: The singular vector in the column space of U, representing the direction in the subspace U, corresponding to the same principal angle.
To incorporate orientation into the score, orientation factor (*λ*) is developed to evaluate the relative alignment or opposition of *qₘᵢₙ* and *pₘᵢₙ* by calculating their dot product. $λ = sgn \left({q}_{min}^{T}{p}_{min}\right)$ where:
if ${q}_{min}^{T} {p}_{min} > 0$, *qₘᵢₙ* and *pₘᵢₙ* point in similar directions, indicating aligned subspaces (+1).
if ${q}_{min}^{T} {p}_{min} < 0$, *qₘᵢₙ* and *pₘᵢₙ* point in opposing directions, indicating contrasting subspace orientations (-1).
A positive value indicates a similar flavor profile, while a negative value signifies a contrasting flavor profile between the two ingredients.

To assess spatial proximity within the flavor subspaces, the centroids of each flavor subspace is calculated and measured the Euclidean distance between them. This involved computing the centroid *Cᵥ* and *Cᵤ* for flavor space of ingredients 1 and 2 respectively. ${C}_{v} = \begin{matrix}1 \\ n\end{matrix} {\sum }_{i = 1}^{n} {v}_{i}$ ${C}_{u} = \begin{matrix}1 \\ m\end{matrix} {\sum }_{j = 1}^{m} {u}_{j}$ The Euclidean distance between the flavor spaces is calculated using the centroid for flavor space for ingredients 1 and 2. This Euclidean distance provides the measure of spatial proximity. $d = \left‖{C}_{v}-{C}_{u}\right‖ = \sqrt{{\sum }_{k = 1}^{d} {\left({C}_{{v}_{k}}-{C}_{{u}_{k}}\right)}^{2}}$

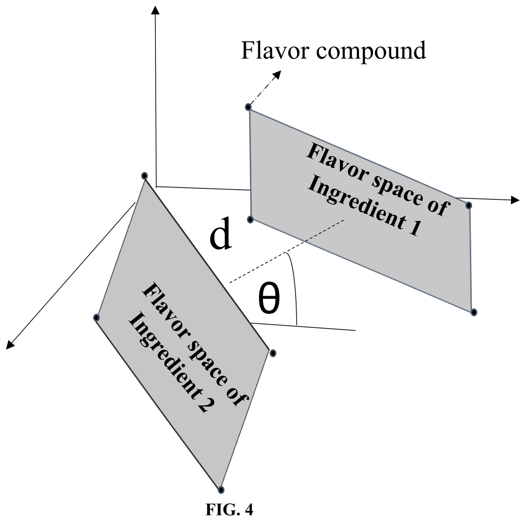

FIG.4 illustrates a flavor space representation of ingredients in three-dimensional space according to some embodiments of the present disclosure. FIG. 4 illustrates the 3D representation of spatial proximity (d) and minimum directional similarity (θ). Using the measures of directional alignment, the orientation factor and the spatial proximity, represented by cos *θ*, *λ* and *d* respectively, the ingredient pairing score is formulated. This score is directly proportional to cos *θ* as it increases with enhanced directional similarity (i.e., as *θ* decreases). Similarly, the score increases as the Euclidean distance between the flavor subspaces decreases. This ingredient pairing score serves as a quantitative measure of flavor compatibility between two ingredients, facilitating the selection of complementary ingredient pairs. $Ingredient pairing score = \frac{λ . cos θ}{1 + d}$ The set of secondary ingredients are selected from the set of ingredients based on the ingredient pairing score between each ingredient amongst the set of ingredients and the primary ingredient.
The step-by-step algorithm for calculating the distance metric and minimum principal angle is provided as below in Algorithm 1,

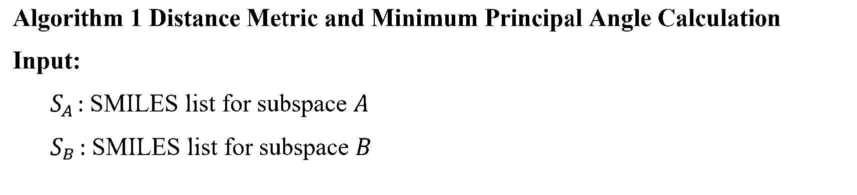

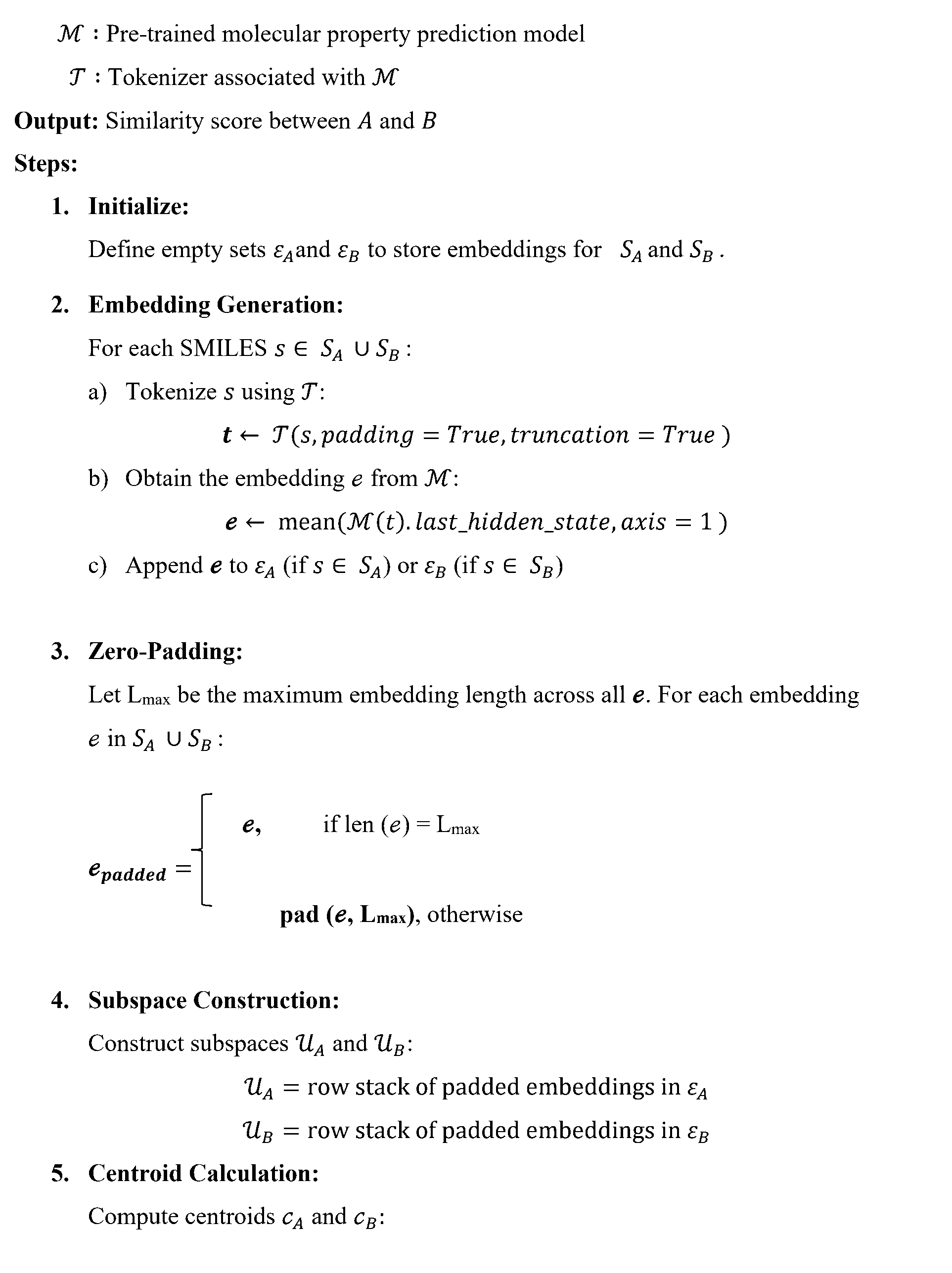

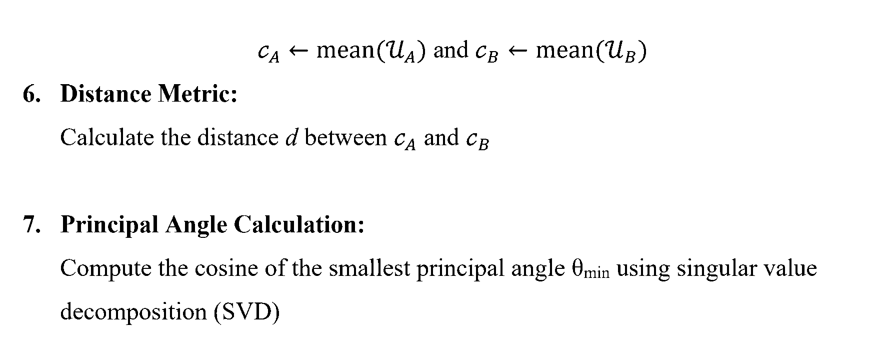

Once the set of secondary ingredients are determined at step 204, then at step 206 of the method 200, one or more hardware processors 102 are configured to generate a recipe comprising a set of cooking instructions from the primary ingredient and the set of secondary ingredients using a text generation model fine-tuned on a recipe dataset based on an optimal token length estimated from an optimal token count per cooking instruction. The recipe generation module utilizes the text generation model specifically trained on a recipe dataset. The dataset used for fine-tuning comprises a plurality of recipes sourced from the recipe dataset. The model takes the list of ingredients received from the ingredient selection module as the input and generates the recipe as the output. For fine-tuning, a subset of the plurality of recipes is randomly extracted from the recipe dataset. This dataset includes information on ingredients, recipe sources, and cooking instructions. The essential data retained for fine-tuning are ingredients and step-by-step instructions. This selection enabled precise alignment of inputs and outputs specific to the recipe generation task. For each recipe, input-output pairs are constructed by prepending the ingredients column with the label "Ingredients:" to form a coherent input text. Similarly, the directions column are prefixed with "Directions:" to construct the desired output text. This process resulted in a refined DataFrame containing two key columns, "input_text" and "output_text", which served as source-target pairs during model training. Table 1 shows an example of a source-target pair present in the Data Frame used for fine-tuning the model. Table 1 contains input and output text constructed for fine-tuning the text generation model (Large language model -LLMs). This structured approach ensured contextual relevance and enhanced the model's ability to generate culinary sequences based on ingredient inputs, facilitating an effective fine-tuning process for recipe generation.

**Table 1**

| **input_text** | **output_text** |
|---|---|
| Ingredients: ["frozen corn", "cream cheese", "butter", "garlic powder", "salt", "pepper"] | Direction: ["In a slow cooker, combine all ingredients". |
| | "Cover and cook on low for 4 hours or until heated through and cheese is melted". "Stir well before serving"." Yields 6 servings."] |

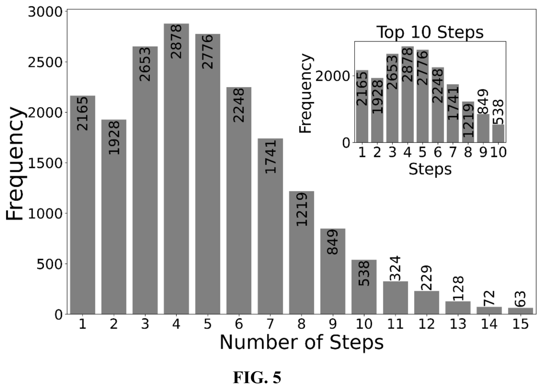

As fine-tuning is a computationally intensive process, the insights gained from data analysis are leveraged to optimize tokenization parameters effectively. FIG. 5 illustrates distribution of the number of cooking instructions within a dataset according to some embodiments of the present disclosure. It is observed that a significant portion of recipes exhibit a mode of five cooking instructions, with approximately 95% of recipes falling within the range of one to ten cooking instructions. The inset figure in FIG.5 illustrates the distribution of recipes with 1 to 10 cooking instructions.

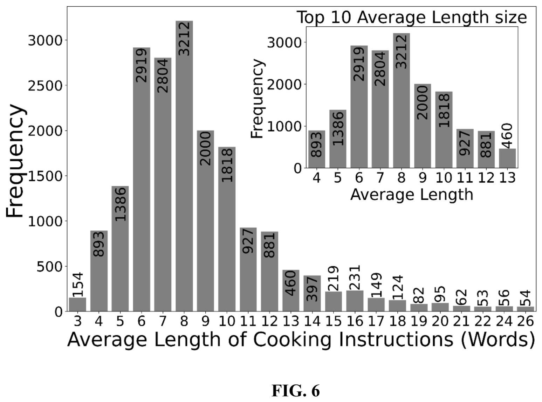

FIG. 6 illustrates the distribution of average cooking instruction length in the dataset according to some embodiments of the present disclosure. This metric helps in identifying trends in instructional detail, which can impact recipe readability and complexity for the end user. The following example illustrates the calculation of average length of cooking instruction:
**Recipe:** *["In a heavy 2-quart saucepan, mix brown sugar, nuts, evaporated milk and butter or margarine.", "Stir over medium heat until mixture bubbles all over top.", "Boil and stir 5 minutes more. Take off heat.", "Stir in vanilla and cereal; mix well.", "Using 2 teaspoons, drop and shape into 30 clusters on wax paper.", "Let stand until firm, about 30 minutes."]* $Averaged length of cooking instruction = \frac{# words in the recipe}{# cookng instructions}$ $Averaged length of cooking instruction = \frac{60}{6} = 10$

Furthermore, most recipes have an average length of cooking instructions between 5 to 15 words, with lengths of 6 to 10 words showing the highest frequency. Consequently, an optimal maximum token length is used by estimating a reasonable token count per cooking instruction step. Assuming an average of 10 to 20 tokens per cooking instruction, the total token count for most recipes fall within the following estimates:
For five cooking instructions: 5 steps x 10 tokens = 50 tokens
For ten cooking instructions: 10 steps x 20 tokens = 200 tokens

While most recipes are expected to lie within the 50 to 200 token range, it is recognized that some outliers may possess additional steps or more elaborate instructions. To accommodate these edge cases without incurring significant computational overhead, a maximum of 256 tokens is used. This length is typically manageable for transformer models and effectively addresses outliers without introducing excessive padding. This parameter not only ensured adequate coverage but also minimized truncation and padding, thereby optimizing both memory usage and processing efficiency during the fine-tuning process. Thus the text generation model tokenizer is set to use the end-of-sequence token for padding, ensuring smooth alignment with the model's architecture. A custom class is developed to manage the dataset effectively. This class tokenized each sample with a maximum sequence length of 256 tokens. Padding and truncation are applied as needed to ensure consistent input dimensions. Optimization is carried out using the AdamW optimizer with a learning rate of 5×10⁻⁵. The learning rate of 5×10⁻⁵ is obtained using hyperparameter tuning. For fine-tuning, a smaller batch size of 1 is employed to effectively manage memory, considering the substantial number of training samples. A single epoch for training is utilized to effectively leverage the substantial size of the dataset while minimizing the risk of overfitting.

### EXPERIMENTAL RESULTS:

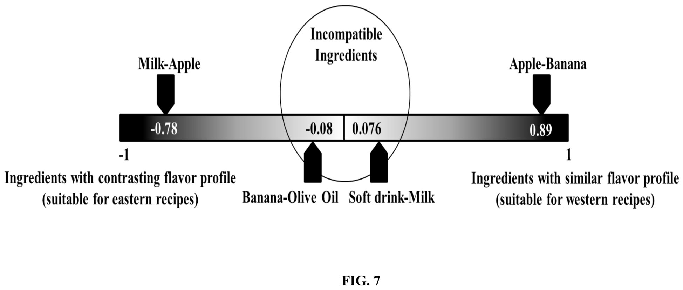

In the ingredient pairing sub-module, pairing scores were evaluated for milk-apple, banana-olive oil, soft drink-condensed milk, and apple-banana. Of the four food pairings analyzed, milk-apple and apple-banana are widely utilized in various recipes, reflecting favorable compatibility. Banana-olive oil and soft drink-milk pairings are considered unsuitable according to literary sources or established culinary knowledge. The milk-apple pairing resulted in a pairing score of -0.78, signifying a significant flavor contrast. Such contrasting flavor profiles are less common in Western cuisine but are frequently utilized in Eastern culinary practices. Apple-banana achieved a high pairing score of 0.89, indicating a complementary flavor profile. This score aligns with its frequent use in culinary applications. In contrast, banana-olive oil and soft drink- condensed milk scored -0.08 and 0.076, respectively. These low scores indicate minimal compatibility, supporting their limited or discouraged use in consumption. The scale presented in FIG.7 illustrates the compatibility of the evaluated ingredient pairings. FIG.7 illustrates flavor compatibility scale representing similar and contrasting flavor profiles according to some embodiments of the present disclosure. Pairings with scores near 0 indicate low similarity or minimal contrasting flavor profiles. Scores approaching -1 signify highly contrasting flavor profiles, while scores closer to 1 represent highly similar flavor profiles.

The method disclosed was used to generate a diabetic friendly recipe. The ingredients shown in Table 2 are based on the user's preference. Among the various ingredients given by the ingredient filtering sub-module, the user chose "Broccoli" as the main ingredient to develop a recipe. Using ingredient pairing sub-module, the user selected the supporting ingredients for the recipe generation. These selected ingredients were used as the input by the recipe generation module to generate a diabetic friendly recipe.

**Table 2**

| **Ingredients** | **Generated Recipe** |
|---|---|
| Broccoli | 1. Cut bell pepper into 1/4-inch slices. |
| | 2. Peel, seed and slice tomatoes. |
| | 3. Sprinkle the vegetables with salt and pepper. |
| Onion | 4. Heat olive oil in large skillet. |
| Bell Pepper | |
| Tomato | 5. Add pepper and onion. |
| Olive Oil | 6. Cook the vegetables over medium-high heat for 3 to 4 minutes or until vegetables are crisp-tender. |
| Salt | |
| Pepper | |
| | 7. Remove from heat. |
| | 8. Stir in boiled broccoli and serve hot. |

For experimental purpose of the disclosed method, ChemBERTa and Bidirectional and Auto-Regressive Transformer (BART) model was used as the pretrained molecular property prediction model and the text generation model respectively. The BART model was trained with RecipeNLG dataset. Though the ChemBERTa model was used only for experimental purposes, any suitable molecular property prediction model can be used to achieve the desired results. Though the BART model was used and trained using RecipeNLG dataset only for experimental purposes, any suitable model can be used and can be trained on similar datasets to achieve the desired results. The BART model was trained on a high-performance system equipped with NVIDIA V100 GPUs, each providing 40 GB of dedicated GPU memory and supported by 60 GiB of system RAM, which was essential for handling the large-scale dataset of approximately 20,000 recipes.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method (200) comprising:
determining, via one or more hardware processors, a set of ingredients from a plurality of ingredients based on a set of macronutrient profiles suitable for one or more medical conditions of a user (202);
determining, via the one or more hardware processors, a set of secondary ingredients from the set of ingredients by evaluating an ingredient compatibility between each ingredient amongst the set of ingredients with a primary ingredient selected from the set of ingredients, wherein the ingredient compatibility is evaluated based on an ingredient pairing score calculated for each ingredient amongst the set of ingredients using a directional alignment measure, an orientation factor, and a spatial proximity measure determined from a molecular embedding matrix represented as a flavor space (204); and
generating, via the one or more hardware processors, a recipe comprising a set of cooking instructions from the primary ingredient and the set of secondary ingredients using a text generation model fine-tuned on a recipe dataset based on an optimal token length estimated from an optimal token count per cooking instruction (206).

2. The processor implemented method as claimed in claim 1, wherein the set of secondary ingredients are determined from the set of ingredients by:
converting, via the one or more hardware processors, a first set of flavor compounds corresponding to the primary ingredient to a first molecular embedding matrix representing a first flavor space using a pre-trained molecular property prediction model;
evaluating, via the one or more hardware processors, the ingredient compatibility between the primary ingredient and each ingredient amongst the set of ingredients by determining the ingredient pairing score; and
selecting, via the one or more hardware processors, the set of secondary ingredients from the set of ingredients based on the ingredient pairing score between each ingredient amongst the set of ingredients and the primary ingredient.

3. The processor implemented method as claimed in claim 2, wherein the ingredient pairing score between the primary ingredient and an ingredient amongst the set of ingredients is determined by:
converting, via the one or more hardware processors, a second set of flavor compounds corresponding to the ingredient to a second molecular embedding matrix representing a second flavor space using the pre-trained molecular property prediction model;
computing, via the one or more hardware processors, the directional similarity measure utilizing a principal angle computed between the first flavor space and the second flavor space;
computing, via the one or more hardware processors, the orientation factor between the flavor space and the second flavor space utilizing a set of singular vectors determined by performing singular value decomposition of a matrix obtained from the first molecular embedding matrix and the second molecular embedding matrix;
computing, via the one or more hardware processors, the spatial proximity measure based on a Euclidean distance measure between a first centroid corresponding to the first flavor space and a second centroid corresponding to the second flavor space; and
determining, via the one or more hardware processors, the ingredient pairing score based on the directional similarity measure, the orientation factor, and the spatial proximity measure.

4. The processor implemented method as claimed in claim 1, wherein the ingredient pairing score is mathematically represented as, $Ingredient pairing score = \frac{λ . cos θ}{1 + d}$ where cos *θ* represents the directional similarity measure, *λ* represents the orientation factor and *d* represents the spatial proximity measure.

5. A system (100), comprising:
a memory (104) storing instructions;
one or more communication interfaces (106); and
one or more hardware processors (102) coupled to the memory (104) via the one or more communication interfaces (106), wherein the one or more hardware processors (102) are configured by the instructions to:
determine a set of ingredients from a plurality of ingredients based on a set of macronutrient profiles suitable for one or more medical conditions of a user;
determine a set of secondary ingredients from the set of ingredients by evaluating an ingredient compatibility between each ingredient amongst the set of ingredients with a primary ingredient selected from the set of ingredients, wherein the ingredient compatibility is evaluated based on an ingredient pairing score calculated for each ingredient amongst the set of ingredients using a directional alignment measure, an orientation factor, and a spatial proximity measure determined from a molecular embedding matrix represented as a flavor space; and
generate a recipe comprising a set of cooking instructions from the primary ingredient and the set of secondary ingredients using a text generation model fine-tuned on a recipe dataset based on an optimal token length estimated from an optimal token count per cooking instruction.

6. The system as claimed in claim 5, wherein the set of secondary ingredients are determined from the set of ingredients by:
converting a first set of flavor compounds corresponding to the primary ingredient to a first molecular embedding matrix representing a first flavor space using a pre-trained molecular property prediction model;
evaluating the ingredient compatibility between the primary ingredient and each ingredient amongst the set of ingredients by determining the ingredient pairing score; and
selecting the set of secondary ingredients from the set of ingredients based on the ingredient pairing score between each ingredient amongst the set of ingredients and the primary ingredient.

7. The system as claimed in claim 6, wherein the ingredient pairing score between the primary ingredient and an ingredient amongst the set of ingredients is determined by:
converting a second set of flavor compounds corresponding to the ingredient to a second molecular embedding matrix representing a second flavor space using the pre-trained molecular property prediction model;
computing the directional similarity measure utilizing a principal angle computed between the first flavor space and the second flavor space;
computing the orientation factor between the flavor space and the second flavor space utilizing a set of singular vectors determined by performing singular value decomposition of a matrix obtained from the first molecular embedding matrix and the second molecular embedding matrix;
computing the spatial proximity measure based on a Euclidean distance measure between a first centroid corresponding to the first flavor space and a second centroid corresponding to the second flavor space; and
determining the ingredient pairing score based on the directional similarity measure, the orientation factor, and the spatial proximity measure.

8. The system as claimed in claim 5, wherein the ingredient pairing score is mathematically represented as, $Ingredient pairing score = \frac{λ . cos θ}{1 + d}$ where cos *θ* represents the directional similarity measure, *λ* represents the orientation factor and *d* represents the spatial proximity measure.

9. One or more non-transitory machine-readable information storage mediums comprising one or more instructions which when executed by one or more hardware processors cause:
determining a set of ingredients from a plurality of ingredients based on a set of macronutrient profiles suitable for one or more medical conditions of a user;
determining a set of secondary ingredients from the set of ingredients by evaluating an ingredient compatibility between each ingredient amongst the set of ingredients with a primary ingredient selected from the set of ingredients, wherein the ingredient compatibility is evaluated based on an ingredient pairing score calculated for each ingredient amongst the set of ingredients using a directional alignment measure, an orientation factor, and a spatial proximity measure determined from a molecular embedding matrix represented as a flavor space; and
generating a recipe comprising a set of cooking instructions from the primary ingredient and the set of secondary ingredients using a text generation model fine-tuned on a recipe dataset based on an optimal token length estimated from an optimal token count per cooking instruction.

10. The one or more non-transitory machine-readable information storage mediums as claimed in claim 9, wherein the set of secondary ingredients are determined from the set of ingredients by:
converting a first set of flavor compounds corresponding to the primary ingredient to a first molecular embedding matrix representing a first flavor space using a pre-trained molecular property prediction model;
evaluating the ingredient compatibility between the primary ingredient and each ingredient amongst the set of ingredients by determining the ingredient pairing score; and
selecting the set of secondary ingredients from the set of ingredients based on the ingredient pairing score between each ingredient amongst the set of ingredients and the primary ingredient.

11. The one or more non-transitory machine-readable information storage mediums as claimed in claim 10, wherein the ingredient pairing score between the primary ingredient and an ingredient amongst the set of ingredients is determined by:
converting a second set of flavor compounds corresponding to the ingredient to a second molecular embedding matrix representing a second flavor space using the pre-trained molecular property prediction model;
computing the directional similarity measure utilizing a principal angle computed between the first flavor space and the second flavor space;
computing the orientation factor between the flavor space and the second flavor space utilizing a set of singular vectors determined by performing singular value decomposition of a matrix obtained from the first molecular embedding matrix and the second molecular embedding matrix;
computing the spatial proximity measure based on a Euclidean distance measure between a first centroid corresponding to the first flavor space and a second centroid corresponding to the second flavor space; and
determining the ingredient pairing score based on the directional similarity measure, the orientation factor, and the spatial proximity measure.

12. The one or more non-transitory machine-readable information storage mediums as claimed in claim 9, wherein the ingredient pairing score is mathematically represented as, $Ingredient pairing score = \frac{λ . cosθ}{1 + d}$ where cos *θ* represents the directional similarity measure, *λ* represents the orientation factor and *d* represents the spatial proximity measure.
